# FASCICULE DE BREVET EUROPEEN

(11) **EP 2 588 073 B1**
(45) Date de publication et mention de la délivrance du brevet: **14.06.2017**
(21) Numéro de dépôt: 11741258.5
(22) Date de dépôt: 28.06.2011
(51) Int. Cl.: A61K 8/60, A61Q 3/02

(54) **COMPOSITION COSMETIQUE POUR LES ONGLES ET L'UTILISATION DE DERIVE D'ISOSORBIDE**
KOSMETIKZUSAMMENSETZUNG FÜR DIE NÄGEL UND VERWENDUNG EINES ISOSORBIDDERIVATS DAFÜR
COSMETIC COMPOSITION FOR THE NAILS AND USE OF AN ISOSORBIDE DERIVATIVE

(30) Priorité: 01.07.2010 FR 1055283
(43) Date de publication de la demande: 08.05.2013
(73) Titulaire: Fiabila, 28130 Maintenon (FR)
(72) Inventeur: NOUGUEREDE, Olivier, F-28130 Hanches (FR); MARTINEZ, Francisco, F-28000 Chartres (FR)
(74) Mandataire: Le Cloirec, Claudine
(86) Numéro de dépôt international: PCT/FR2011/051500
(87) Numéro de publication internationale: WO 2012/001293

(56) Documents cités:
- EP-A1- 2 174 641
- WO-A1-99/45060
- WO-A1-2006/103338
- FR-A1- 2 895 905
- chambres agriculture picardie REPÈRES AGRO-INDUSTRIES/NORD-PAS-DE-CALAIS/PICARDI E, no. 122 septembre 2009 (2009-09), XP002629660, Extrait de l'Internet: URL:http://www.chambres-agriculture-picard ie.fr/fileadmin/documents/publications/eco nomie/AIR122.PDF [extrait le 2011-03-23]

## Description

La présente invention concerne le domaine des compositions pour les ongles, notamment les compositions cosmétiques pour les ongles, tels que les vernis à ongles.

De manière classique, les vernis à ongles renferment comme principaux constituants des solvants organiques ou aqueux, au moins un composé filmogène et un plastifiant, des pigments et/ou des composés colorants. Depuis quelques années, on cherche à formuler des compositions pour les ongles sans phtalates, ni produits de condensation de l'acétaldéhyde, composés qui se sont avérés dangereux pour la santé. On cherche donc à mettre au point de nouveaux plastifiants, moins toxiques et/ou moins volatils.

Par ailleurs la tendance actuelle, dans tous les secteurs, et notamment dans le domaine des cosmétiques, est de recourir de plus en plus à des produits dits naturels, en particulier issus de substances végétales.

Cependant, le formulateur est confronté à de multiples difficultés et contraintes. En premier lieu, les contraintes sont liées à la nature spécifique du support qu'est un ongle. En effet, l'ongle est un support "vivant" qui interagit avec son environnement. Il contient de 15 à 20 % d'eau selon la température, l'humidité ambiante et les activités de la personne. Toute composition de vernis doit être adaptée à ce support spécifique en termes d'adhérence, de perméabilité à l'oxygène et à la vapeur d'eau, avec un compromis dureté/flexibilité. Il ne s'agit pas d'un support inerte, l'une des faces de l'ongle est à 35°C environ et l'autre est exposée à des températures pouvant aller de - 20°C à +50°C selon la saison, le lieu, les activités...

De plus, chaque changement de constituant ou chaque modification de la concentration d'un constituant de la composition a une influence sur les propriétés recherchées pour les vernis, telles que l'adhérence, la brillance, et la dureté mais aussi la flexibilité.

Par exemple, depuis quelques années, l'acétyltributylcitrate (ATC) (cité notamment dans US 5,227,155) a remplacé presque exclusivement les phtalates utilisés initialement comme plastifiants. On a cependant observé que l'ATC confère au vernis un moins bon brillant que les phtalates.

Par ailleurs, il a été constaté que certains plastifiants, dits naturels, favorisent soit une reprise en eau du vernis, c'est-à-dire présentent l'inconvénient de conduire, après séchage du vernis sur l'ongle, à une absorption d'eau (par exemple lorsque le sujet se baigne, se lave, ou a les mains dans l'eau pour des activités ménagères...) soit une dégradation par migration dans l'eau de constituants hydrophiles du vernis ; c'est le cas, par exemple, des plastifiants à base de carbonate, tel que le carbonate de glycérol (WO2007/080172). Cette absorption d'eau qui provoque un gonflement du vernis, ou cette dégradation, conduisent à une diminution de sa résistance et une perte d'adhérence. Ces conséquences sont très préjudiciables au maintien du vernis sur l'ongle.

Des constatations similaires ont été faites avec des vernis à ongles renfermant de l'ATC, dans des régions à forte hygrométrie. Ces vernis présentaient des défauts d'adhérence, qui se sont avérés être provoqués par l'absorption de l'humidité ambiante.

Un but de la présente invention est donc de proposer un vernis à ongles qui pallie les inconvénients ci-dessus, en remplissant les critères requis d'adhérence et de brillance, et présentant un bon compromis dureté/flexibilité.

Le constituant qui joue un rôle majeur dans l'obtention de ces propriétés étant le plastifiant, un autre but de l'invention est d'utiliser un plastifiant d'origine naturelle, ne provoquant pas de gonflement, c'est-à-dire à absorption d'eau réduite, de manière à conférer au vernis appliqué sur l'ongle une faible reprise en eau après séchage.

Ces buts sont atteints par le vernis à ongles selon la présente invention, comprenant de la cellulose, caractérisée en ce qu'il renferme un diester d'isosorbide, plastifiant d'origine naturelle, dérivé du sorbitol de formule (I) ci-après.

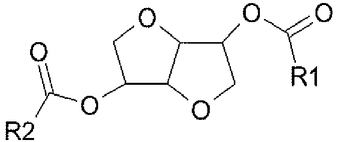

En effet, le demandeur a notamment découvert, de manière surprenante que les diesters d'isosorbide pouvaient remplir cette fonction de plastifiant dans des compositions de vernis, notamment en conférant à ces vernis des propriétés supérieures aux compositions renfermant des plastifiants de l'art antérieur.

L'isosorbide est un produit obtenu par déshydration d'un dérivé du glucose, le sorbitol, qui peut notamment être extrait de baies de sorbier ou de céréales. Le diester est avantageusement produit par réaction entre un acide gras d'origine végétale et l'isosorbide.

Dans la formule (I) ci-dessus, les radicaux R1 et R2 sont des radicaux alkyles identiques en C₆ à C₁₂, linéaires ou ramifiés.

Selon l'invention, le plastifiant peut être choisi parmi les composés suivants : le dihexanoate d'isosorbide, le diheptanoate d'isosorbide, le dioctanoate d'isosorbide, le didécanoate d'isosorbide, le didodécanoate d'isosorbide, le di(2-éthylhexanoate)d'isosorbide, ou un mélange de ceux-ci.

Ces plastifiants répondent au problème technique évoqué ci-dessus en permettant d'élaborer des compositions cosmétiques filmogènes pour les ongles qui présentent un bon compromis dureté/flexibilité avec une bonne compatibilité avec les autres constituants de la formule. On remarque également une adhésion correcte, ainsi qu'une très faible absorption d'eau, des vernis formulés avec de tels plastifiants.

Ces diesters d'isosorbide présentent également l'avantage d'être des produits issus de substances naturelles et sont des produits biodégradables. En effet, l'isosorbide est obtenu par déshydratation du sorbitol, lui-même obtenu ici à partir de céréales. Les acides gras utilisés pour la fabrication du diester sont issus d'huiles végétales.

Le plastifiant est avantageusement présent dans la composition cosmétique pour les ongles dans une concentration comprise entre 0,1 et 30 % en poids, préférentiellement de 3 à 20 %, de préférence encore entre 5 et 15 % en poids et de préférence encore entre 9 et 11 % en poids.

Ce vernis peut être employé comme base pour vernis (appelée base coat), comme vernis de maquillage des ongles, comme composition de finition (appelée top coat) pour les ongles, ou comme composition de soin cosmétique des ongles. Cette composition peut s'appliquer sur l'ongle humain ou sur des faux ongles.

Ce vernis renferme un polymère filmogène principal dérivé de la cellulose, tel que la nitrocellulose, et peut renfermer aussi l'acétate de cellulose, l'acétobutyrate de cellulose, l'acétopropionate de cellulose, ou un mélange de ceux-ci. Il peut renfermer également un ou plusieurs polymères filmogènes secondaires tels que les résines polyester, alkyde, acrylique, époxy-tosylamide, polyuréthanne, polyamide, ..., des agents épaississants minéraux ou organiques, tels que des silices, des argiles modifiées, des matières colorantes tels que des pigments ou nacres, des additifs tels que des agents anti UV, des agents mouillants, des agents d'étalement et/ou de glissance, des agents hydratants, des actifs (Vitamine B5, E, C, acides aminés, huiles, ...) et/ou des parfums.

Par polymère(s) filmogène(s) secondaire(s) on entend ici un ou plusieurs polymère(s) filmogène(s) en moindre proportion pondérale que le polymère filmogène principal.

La présente invention concerne également l'utilisation de diester d'isosorbide en tant que plastifiant dans des compositions cosmétiques pour les ongles, notamment les compositions cosmétiques comprenant un composé filmogène dérivé de la cellulose.

La présente invention sera mieux comprise à l'aide de la description qui suit d'exemples mentionnés à titre d'illustration.

### EXEMPLES

Différents plastifiants ont été testés :
Exemple 1 - Le dioctanoate d'isosorbide, produit commercialisé par la Société ROQUETTE sous la dénomination Polysorb ID37.
Exemple 2 (comparatif) - L'acétyl tributyl citrate (tel qu'utilisé dans l'art antérieur - voir US 5,227,155)
Exemple 3 (comparatif) - Le diéthylène glycol dibenzoate
Exemple 4 (comparatif) - Le glyceryl tribenzoate
Exemple 5 (comparatif) - Le carbonate de glycérol (tel que cité dans la demande WO 2007/080172)

La formule générale utilisée pour comparer les différents plastifiants était la suivante :

**Tableau 1**

| **Constituant** | **% en poids** |
|---|---|
| Acétate d'éthyle | 20,77 |
| Acétate de butyle | 40,50 |
| Nitrocellulose (70 % dans isopropanol) | 18,00 |
| Plastifiant | 10,00 |
| Résine polyester (Anhydride trimellitique/Acide adipique/Néopentyl glycol) à 70% dans l'acétate de butyle | 9,00 |
| Acide phosphorique | 0,03 |
| Bentonite | 1,50 |
| Diacétone alcool | 0,20 |
| **TOTAL** | **100,00** |

### (Les pourcentages sont exprimés en poids total de la composition)

Cette formule a été optimisée en termes de performances (voir ci-dessous). La majeure partie des constituants provient du domaine végétal. La nitrocellulose pourrait également être fournie à 70 % dans l'éthanol, ce qui permettrait d'augmenter le pourcentage de matières premières provenant du végétal dans la formule finale.

Les tests réalisés sur ces différents vernis sont les suivants (les valeurs souhaitées des différents paramètres mesurés sont notés entre parenthèses) :
- Brillance : Sur une plaque Leneta, on applique un film de vernis de 100µm. On fait sécher, puis on mesure, au moyen d'un brillancemètre, le brillant à un angle de 60° (une valeur supérieure à 80° est souhaitée).
- Flexibilité : Sur une plaque en aluminium recouverte d'un vernis de 300µm humide, on réalise un emboutissage lent et on mesure la profondeur de la pénétration (ISO1520) (une valeur supérieure à 3,0 est souhaitée, de préférence supérieure à 4,0).
- Dureté : Dureté Persoz sur une plaque de verre recouverte d'un vernis de 100µm humide (ISO1522) (une valeur minimale de 180 est nécessaire, préférentiellement 220).

- Immersion dans l'eau : Un film préalablement séché est immergé durant 24h dans de l'eau à une température de 25°C. La variation de masse du film avant et après immersion est mesurée (une valeur la plus faible possible est souhaitée pour limiter le gonflement qui provoquerait un ramollissement et un décollement du vernis).
- Adhérence : "Cross hatch test" réalisé sur plaque de verre. La note 0 correspond à l'absence de perte d'adhésion. La note 5 correspond à la perte totale d'adhésion (une valeur comprise entre 0 et 1 est indispensable).
- Extrait sec : Dans une coupelle, on pèse entre 0,5g et 1g de vernis. On place ensuite cette plaque à l'étuve durant 3 heures à 100°C. On pèse la coupelle après son passage à l'étuve et on calcule l'extrait sec du vernis (les valeurs habituelles sont généralement comprises entre 30 et 32 % en poids d'extrait sec).

Les résultats obtenus sont regroupés dans le tableau 2 qui suit.

**Tableau 2**

| | **Ex.1** | **Ex.2 (Comp)** | **Ex.3 (Comp)** | **Ex.4 (Comp)** | **Ex.5 (Comp)** |
|---|---|---|---|---|---|
| Extrait sec (%) | 30,7 | 31,2 | 31,1 | 32,2 | 30,4 |
| Dureté (s) | 248 | 229 | 220 | 246 | 60 |
| Flexibilité (mm) | 4,6 | 5,3 | 4,6 | 1,5 | 5,9 |
| Brillance (60°, UB) | 88,5 | 83,5 | 90,3 | 90,3 | 84,4 |
| Adhérence | 0,1 | 0,1 | 2 | 0,1 | 5 |
| Evolution massique après immersion (%) | + 2,4 | + 4,2 | + 2,6 | + 2 | - 14,4 |

On constate parmi les plastifiants qu'aucun des plastifiants des exemples comparatifs 2, 3, 4 et 5 n'est satisfaisant.

En effet, l'acétyl tributylcitrate (exemple 2 comparatif) présente une brillance inférieure et une reprise en eau supérieure par rapport au dioctanoate d'isosorbide.

L'exemple 3 comparatif montre que le diéthylèneglycol dibenzoate présente une adhérence insuffisante.

Le tribenzoate de glycérol (exemple 4 comparatif) présente une flexibilité très inférieure à celle des autres plastifiants, et qui est très insuffisante pour une utilisation dans une telle composition de vernis.

Quant au carbonate de glycérol, il est également à exclure puisqu'il présente une dureté très basse ainsi qu'une plus faible brillance que les autres plastifiants. Son adhérence est également très insuffisante avec une perte totale d'adhésion.

En outre, il présente une dégradation importante après immersion dans l'eau, avec une perte massique importante.

On remarque donc les excellentes performances du dioctanoate d'isosorbide par rapport aux autres plastifiants dans les mêmes conditions opératoires.

### Exemple 6

Dans cet exemple, on a fait varier la concentration de plastifiant de la formule de l'exemple 1. Les résultats des formules A, B et C sont reportés dans le tableau 3 ci-dessous.

**Tableau 3**

| **Formules** | **A** | **B** | **C** |
|---|---|---|---|
| Acétate d'éthyle | 20,77 | 20,77 | 20,77 |
| Acétate de butyle | 38,5 | 40,5 | 42,5 |
| Nitrocellulose (70% dans isopropanol) | 18 | 18 | 18 |
| Dioctanoate d'isosorbide | 12 | 10 | 8 |
| Résine polyester (Anhydride trimellitique/Acide adipique/Néopentyl glycol) à 70% dans l'acétate de butyle | 9 | 9 | 9 |
| Acide phosphorique | 0,03 | 0,03 | 0,03 |
| Bentonite | 1,5 | 1,5 | 1,5 |
| Diacétone alcool | 0,2 | 0,2 | 0,2 |

Seule la concentration en solvant a été modifiée pour tenir compte des différentes variations de pourcentage en poids du plastifiant.

Les résultats des différents tests obtenus avec ces trois formules A, B et C sont reportés dans le tableau 4 ci-dessous.

**Tableau 4**

| | **Essai A** | **Essai B** | **Essai C** |
|---|---|---|---|
| Extrait sec (%) | 32,5 | 30,7 | 28,6 |
| Dureté (s) | 189 | 248 | 309 |
| Flexibilité (mm) | 5,7 | 4,6 | 3,1 |
| Brillance (60°, UB) | 88,6 | 88,5 | 87,5 |
| Adhérence | 0-1 | 0-1 | 0-1 |

On voit donc que, dans les formules décrites dans le tableau 1, la quantité optimale de dioctanoate d'isosorbide est de l'ordre de 10 % en poids. Les concentrations plus fortes conduisent à une dureté plus faible et des concentrations inférieures conduisent à une moindre flexibilité.

Toutefois, cette concentration optimale peut varier en fonction de la nature et de la concentration des autres matières premières présentes dans le vernis à ongles.

L'invention a été décrite selon un mode de réalisation particulier, il est évident qu'elle n'y est nullement limitée et qu'elle comprend tous les équivalents techniques décrits entrant dans le cadre des revendications. La composition de l'exemple B comprend 84 % de matière première d'origine naturelle, en utilisant des solvants d'origine végétale. Ce taux pourrait facilement monter à 92 % si une nitrocellulose diluée dans de l'éthanol d'origine végétale était utilisée.

## Revendications

1. Vernis à ongles, comprenant de la nitrocellulose, **caractérisé en ce qu'**il renferme un diester d'isosorbide, plastifiant d'origine naturelle, dérivé du sorbitol, de formule (I) ci-après : 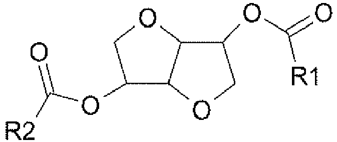 R1 et R2 étant des radicaux alkyles identiques en C₆ à C₁₂ linéaires ou ramifiés.

2. Vernis selon la revendication 1, **caractérisé en ce que** le plastifiant est choisi parmi les composés suivants : le dihexanoate d'isosorbide, le diheptanoate d'isosorbide, le dioctanoate d'isosorbide, le didécanoate d'isosorbide, le didodécanoate d'isosorbide, le di(2-éthylhexanoate)d'isosorbide, ou un mélange de ceux-ci.

3. Vernis selon l'une quelconque des revendications précédentes, **caractérisé en ce qu'**il renferme au moins un solvant, de la nitrocellulose, en tant que polymère filmogène principal, et un ou plusieurs polymère(s) filmogène(s) secondaire(s).

4. Vernis selon l'une quelconque des revendications précédentes, **caractérisé en ce que** le plastifiant est présent dans une concentration comprise entre 0,1 et 30 % en poids, de préférence entre 3 et 20 % en poids.

5. Vernis selon l'une quelconque des revendications précédentes, **caractérisé en ce qu'**il s'agit d'une base pour vernis, un vernis de maquillage pour les ongles, une composition de finition pour les ongles ou une composition de soin cosmétique des ongles.

6. Vernis selon l'une quelconque des revendications précédentes, **caractérisé en ce qu'**il renferme des agents épaississants minéraux ou organiques, des pigments ou nacres, des agents anti UV, des agents mouillants, des agents d'étalement et/ou de glissance, des agents hydratants, des vitamines et/ou des parfums.

7. Utilisation, en tant que plastifiant dans des vernis à ongles comprenant de la nitrocellulose, d'un diester d'isosorbide dérivé du sorbitol, de formule (I) ci-après : 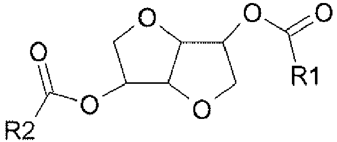 R1 et R2 étant des radicaux alkyles identiques en C₆ à C₁₂ linéaires ou ramifiés.

## Patentansprüche

1. Nagellack, umfassend Nitrocellulose, **dadurch gekennzeichnet, dass** er einen Isosorbiddiester, Weichmacher natürlichen Ursprungs, Derivat des Sorbitols, der folgenden Formel (I) umfasst: 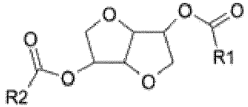 wobei R1 und R2 identische lineare oder verzweigte C₆-C₁₂-Alkylradikale sind.

2. Lack nach Anspruch 1, **dadurch gekennzeichnet, dass** der Weichmacher aus den folgenden Verbindungen ausgewählt ist: dem Isosorbiddihexanoat, dem Isosorbiddiheptanoat, dem Isosorbiddioctanoat, dem Isosorbiddidecanoat, dem Isosorbiddidodecanoat, dem Isosorbiddi(2-ethylhexanoat) oder einem Gemisch derselben.

3. Lack nach einem der vorangehenden Ansprüche, **dadurch gekennzeichnet, dass** er mindestens ein Lösungsmittel, Nitrocellulose, als hauptsächliches filmbildendes Polymer und ein oder mehrere sekundäre filmbildende Polymere umfasst.

4. Lack nach einem der vorangehenden Ansprüche, **dadurch gekennzeichnet, dass** der Weichmacher in einer Konzentration zwischen 0,1 und 30 Gew.-%, vorzugsweise zwischen 3 und 20 Gew.-%, inklusive vorhanden ist.

5. Lack nach einem der vorangehenden Ansprüche, **dadurch gekennzeichnet, dass** es sich um eine Lackbasis, einen Make-up-Lack für die Nägel, eine Finishzusammensetzung für die Nägel oder eine kosmetische Pflegezusammensetzung der Nägel handelt.

6. Lack nach einem der vorangehenden Ansprüche, **dadurch gekennzeichnet, dass** er mineralische oder organische Verdickungsmittel, Pigmente oder Perlmutt, Anti-UV-Mittel, Befeuchtungsmittel, Verteilungs- und/oder Rutschmittel, Feuchtigkeitsmittel, Vitamine und/oder Duftstoffe umfasst.

7. Verwendung als Weichmacher in Nagellacken, umfassend Nitrocellulose, eines Isosorbiddiesters als Derivat des Sorbitols, der folgenden Formel (I): 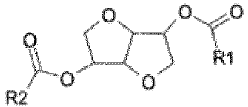 wobei R1 und R2 identische lineare oder verzweigte C₆-C₁₂-Alkylradikale sind.

## Claims

1. A nail varnish, comprising nitrocellulose **characterized in that** it contains an isosorbide diester, a plasticizer of natural origin, derived from sorbitol, of formula (1) hereafter: 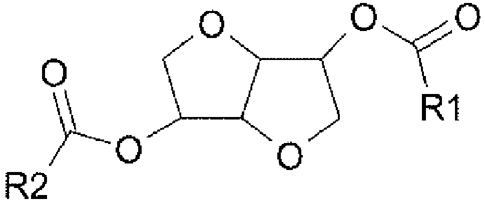 R1 and R2 being identical linear or branched C₆-C₁₂ alkyl radicals.

2. The varnish according to claim 1, **characterized in that** the plasticizer is selected from among the following compounds: isosorbide dihexanoate, isosorbide diheptanoate, isosorbide dioctanoate, isosorbide didecanoate, isosorbide didodecanoate, isosorbide di(2-ethylhexanoate), or mixture thereof.

3. The varnish according to any of the preceding claims, **characterized in that** it contains at least one solvent, nitrocellulose, as a main film-forming polymer, and one or several secondary film-forming polymers.

4. The varnish according to any of the preceding claims, **characterized in that** the plasticizer is present in a concentration comprised between 0.1 and 30% by weight, preferably between 3 and 20% by weight.

5. The varnish according to any of the preceding claims, **characterized in that** this is a base for a varnish, a varnish for making up nails, a finishing composition for nails, or a cosmetic care composition for nails.

6. The varnish according to any of the preceding claims, **characterized in that** it contains mineral or organic thickeners, pigments or mother-of-pearls, anti-UV agents, wetting agents, spreading and/or slipping agents, moisturizers, vitamins and/or perfumes.

7. The use, as a plasticizer in nail varnishes comprising nitrocellulose, of an isosorbide diester derived from sorbitol, of formula (I) hereafter: 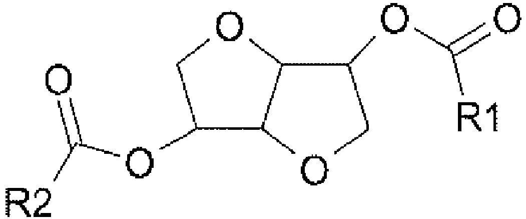 R1 and R2 being identical linear or branched C₆-C₁₂ alkyl radicals.
